(19) Europäisches Patentamt / European Patent Office / Office européen des brevets

(11) **EP 3 658 887 B1**

(12) **EUROPEAN PATENT SPECIFICATION**

(45) Date of publication and mention
of the grant of the patent:
**25.08.2021 Bulletin 2021/34**

(21) Application number: **18746876.4**

(22) Date of filing: **24.07.2018**

(51) Int Cl.:
*G01N 9/16* (2006.01)  *G01N 9/26* (2006.01)

(86) International application number:
**PCT/EP2018/070031**

(87) International publication number:
**WO 2019/020621 (31.01.2019 Gazette 2019/05)**

(54) **METHODS AND SYSTEM FOR MEASURING DENSITY OF FRESH CONCRETE**

VERFAHREN UND SYSTEM ZUR MESSUNG DER DICHTE VON FRISCHBETON

PROCÉDÉS ET SYSTÈME DE MESURE DE DENSITÉ DE BÉTON FRAIS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.07.2017 US 201762538241 P**

(43) Date of publication of application:
**03.06.2020 Bulletin 2020/23**

(73) Proprietor: **Command Alkon Incorporated
Birmingham, AL 35243 (US)**

(72) Inventor: **BEAUPRE, Denis
Sainte-Catherine-de-la-Jacques-Cartier,
Québec G3N 0V6 (CA)**

(74) Representative: **Plasseraud IP
66, rue de la Chaussée d'Antin
75440 Paris Cedex 09 (FR)**

(56) References cited:
**WO-A1-2011/042880    WO-A1-2014/138968
WO-A1-2016/023119    US-A- 4 900 154**

**Description**

**FIELD**

The improvements generally relate to the field of concrete production, and more particularly refers to measuring density of fresh concrete inside a drum of a mixer truck.

**BACKGROUND**

It is well known in concrete industry that density of fresh concrete is affected by concrete composition and air content.

Density is usually calculated by dividing a known volume of material by its weight. For a given composition, the measured or calculated density of fresh concrete can be compared to the theoretical density without considering the presence of air to calculate the theoretical air content. The measure of density usually requires the use of a container known volume that is filled with the fresh concrete and the weight of the concrete is determined by discounting the weight of the container.

International patent publication no. WO 2014/138968 describes a technique for estimating density of fresh concrete inside a drum of a mixer truck. In this technique, the density of the fresh concrete is determined from force pressures using a pressure/force measurement probe. In this technique, pressure or force values indicative of the corresponding pressure or force exerted on the probe and the associated probe angle are recorded for a plurality of circumferential positions of the probe along its movement path in the fresh concrete. Then, a valid data set is extracted from the recorded data so as to calculate a linear relationship which is best fitted to the valid data set. This best-fit linear relationship curve is proportional to the concrete density in accordance with a coefficient which is a trigonometric factor of the fluid and the geometrical and mechanical features of the pressure sensor. Henceforth, the proportionality coefficient can be calibrated against measurements of slope for known density.

Although existing technique for measuring the density of fresh concrete are satisfactory to a certain degree, there remains room for improvement.

**SUMMARY**

There is described a method and a system for measuring density of fresh concrete inside a drum of a mixer truck using a probe. The drum of the mixer truck can rotate about a rotation axis which is at least partially horizontally-oriented relative to the vertical. The probe is mounted inside the drum so as to extend in a radial orientation of the drum. Accordingly, upon rotation of the drum, the probe is moved circumferentially as the drum rotates. When fresh concrete is present inside the drum, and when the probe is submerged and moved in the fresh concrete by the rotation of the drum, a normal pressure is imparted on the probe including normal contributions of buoyancy due to a volume of the probe and of resistance due to the movement of the probe in the fresh concrete.

Accordingly, it was determined that a density of the fresh concrete can be determined based on a first pressure value indicative of a normal pressure exerted on the probe at a first circumferential position of the drum and on a second pressure value indicative of a normal pressure exerted on the probe at a second circumferential position different from the first circumferential position. More importantly, it was found that the density value can be determined based on the volume of the probe and on a difference between the first pressure value and the second pressure value. In some embodiments, the difference is compensated by a trigonometric factor corresponding to a difference between the sinus of the first circumferential position and the sinus of the second circumferential position. In further embodiments, the first and second circumferential positions are preferably chosen so as to be circumferentially away from the bottom of the drum to avoid potential discrepancies in the measured pressure values when the probe is in the vicinity of the bottom of the drum.

In accordance with one aspect, there is provided a method for determining density of fresh concrete inside a drum of a mixer truck using a probe, according to claim 1.

In accordance with another aspect, there is provided a system for determining density of fresh concrete inside a drum of a mixer truck, according to claim 10.

Many further features and combinations thereof concerning the present improvements will appear to those skilled in the art following a reading of the instant disclosure.

**DESCRIPTION OF THE FIGURES**

In the figures,

Fig. 1 is a side elevation view of an example of a system for determining the density of fresh concrete inside a drum of a mixer truck, in accordance with an embodiment;

Fig. 2 is a sectional view taken along line 2-2 of Fig. 1;

Fig. 3 is an example of a graph showing a normal contribution of gravity measured by the probe during a rotation of a drum;

Fig. 4 is an example of a graph showing, for two different rotation speeds of a drum, normal contributions of buoyancy measured by the probe when submerged into water during a rotation of a drum at a corresponding one of the two rotation speeds of the drum, after mathematically discounting the normal contribution of gravity;

Fig. 5 is an example of a graph showing, for two different rotation speeds of a drum, normal contributions of resistance exerted on the probe by the movement of the probe into the fresh concrete during a rotation of a drum at a corresponding one of the two rotation speeds of the drum;

Fig. 6 is an example of a graph showing, for two different rotation speeds of a drum, normal contributions of buoyancy and resistance measured during a rotation of a drum at a corresponding one of the two rotation speeds of the drum;

Fig. 7 is an example of a graph showing, for two different rotation speeds of a drum, experimental normal contributions of buoyancy and resistance measured during a rotation of a drum at a corresponding one of the two rotation speeds of the drum, with discrepancies for pressure values measured in the vicinity of the bottom of the drum;

Fig. 8 is a sectional view of a drum of a mixer truck, showing exemplary circumferential ranges, in accordance with an embodiment;

Fig. 9 is an example of a graph showing, for two different rotation speeds of a drum, experimental normal contributions of buoyancy and resistance measured during a rotation of a drum at a corresponding one of the two rotation speeds of the drum, with discrepancies for pressure values measured in the vicinity of the bottom of the drum and average pressure values for some of the circumferential ranges of Fig. 8; and

Fig. 10 is an example of a graph showing average pressure values as a function of the rotation speed of the drum, in accordance with an embodiment.

## DETAILED DESCRIPTION

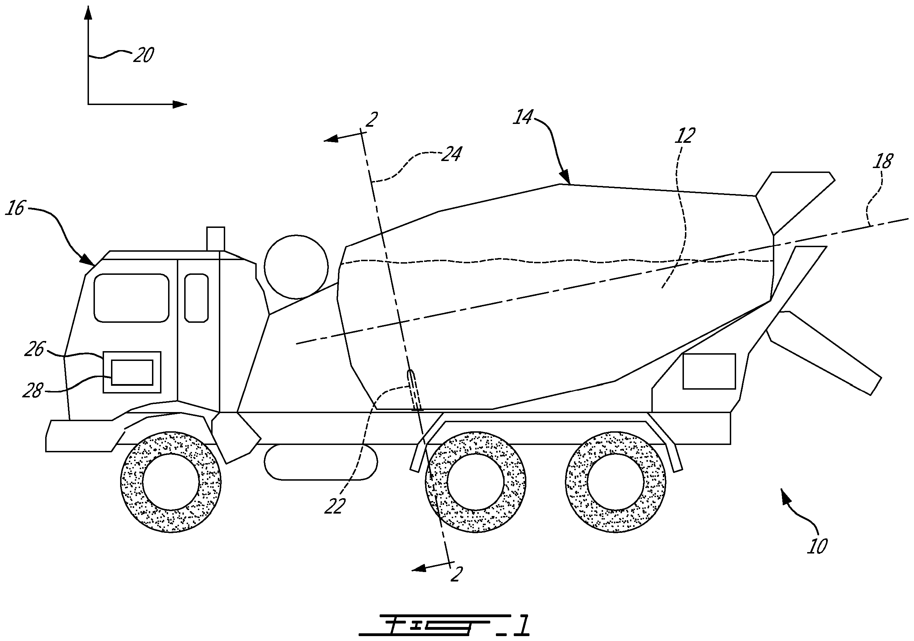

Fig. 1 shows an example of a system 10 for determining density of fresh concrete 12 inside a drum 14 of a mixer truck 16. As shown, the drum 14 has a rotation axis 18 which is at least partially horizontally-oriented relative to the vertical 20.

As depicted in this example, the system 10 has a probe 22 which is mounted inside the drum 14 and extends in a radial orientation 24 of the drum 14. The probe 22 is configured to measure pressure values as the probe 22 is moved circumferentially in the fresh concrete 12 by the rotation of the drum 14 about the rotation axis 18. As the probe 22 is so moved, it reaches a plurality of circumferential positions, which are associated to corresponding ones of the pressure values measured by the probe 22. A potential example of the probe 22 is described in international patent publication no. WO 2011/042880.

The system has a computing device 26 which is communicatively coupled with the probe 22 so that the computing device 26 can receive the pressure values measured by the probe 22 and the corresponding circumferential positions $\theta$. The communication between the computing device 26 and the probe 22 can be provided by a wireless connection, a wired connection, or a combination thereof.

As will be described below, a density value D of the fresh concrete 12 can be determined by the computing device 26 based on at least two received pressure values and their corresponding circumferential positions $\theta$ and on at least one parameter which can depend on a volume of the probe 22, as will be described herebelow.

The system 10 has a user interface 28 which is communicatively coupled with the computing device 26 and configured to display the density value D of the fresh concrete 12 once determined. The density value D can be displayed in real time on the user interface 28 or be stored on a memory of the computing device 26 for display at a later time or on another user interface.

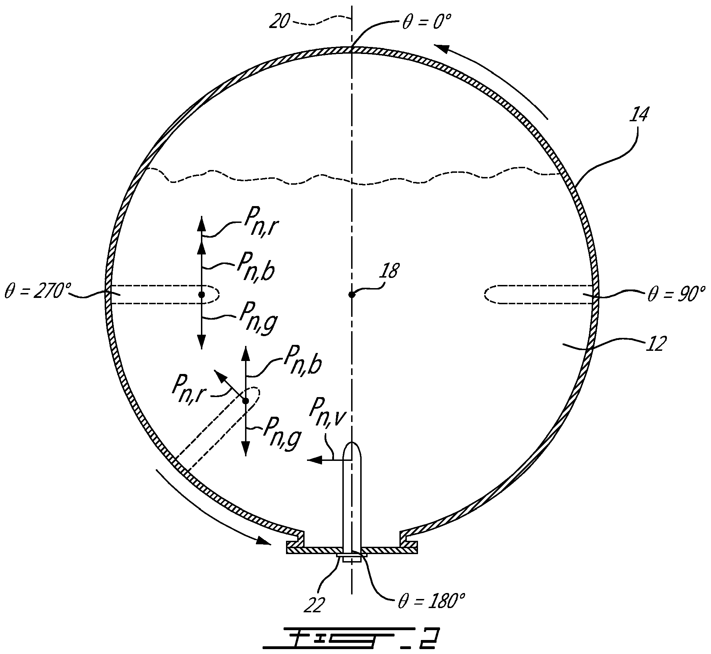

As best seen in Fig. 2, the probe 22 extends in a radial orientation 20 of the drum and reaches a plurality of circumferential positions $\theta$ as the drum 14 rotates about the rotation axis 18. More specifically, in this illustrated example, the probe 22 is at a circumferential position θ of 0° when the probe 22 is at the top of the drum 14, the probe 22 is at a circumferential position of 90° when the probe 22 is at the right of the drum 14, the probe 22 is at a circumferential position of 180° when the probe 22 is at the bottom of the drum 14, and the probe 22 is at a circumferential position of 270° when the probe 22 is at the left of the drum 14. Such definition of the circumferential positions θ is exemplary only as the circumferential positions θ could have been defined otherwise depending on the embodiment.

At each of the circumferential positions θ, the probe 22 measures a pressure value and transmits the pressure value and the corresponding circumferential position θ to the computing device 26. The pressure values that are measured are oriented in a normal orientation with respect to the probe 22. Such pressure values can be referred to as "normal pressure values" and can include a normal contribution $P_{n,g}$ imparted on the probe 22 by gravity due to a weight of the probe 22, a normal contribution $P_{n,b}$ imparted on the probe 22 by buoyancy due to a volume of the probe 22 when the probe 22 is submerged in the fresh concrete 12 and a normal contribution $P_{n,r}$ imparted on the probe 22 by resistance due to the movement of the probe 22 in the fresh concrete 12 by the rotation of the drum 14. Fig. 2 shows normal contributions $P_{n,g}$, $P_{n,b}$, $P_{n,r}$ by way of force vectors acting on the probe 22 when positioned at different circumferential positions.

The gravity depends on a mass m of the probe 22 and on the gravitational acceleration g, and acts on the probe 22 along the vertical 20. Accordingly, the normal contribution $P_{n,g}$ of the gravity exerted on the probe 22 varies with its circumferential position θ. For instance, when the probe 22 is horizontally-oriented, e.g., when the probe 22 is at the circumferential position θ = 90° or θ = 270°, the normal contribution $P_{n,g}$ of the gravity is either maximal or minimal, as the gravity pulls the probe 22 toward the ground and creates a downward pressure on it. In contrast, when the probe 22 is vertically-oriented, e.g., when the probe 22 is at the bottom of the drum 14 so that its circumferential position is θ = 180°, the normal contribution $P_{n,g}$ of the gravity is null.

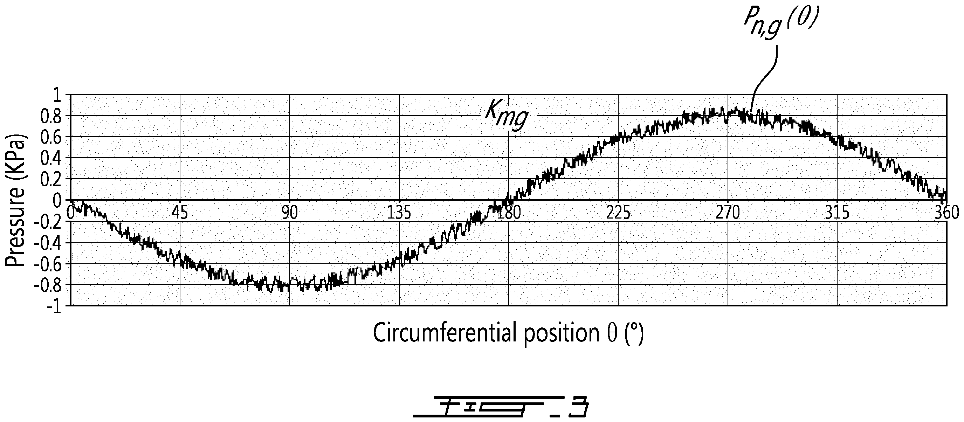

Fig. 3 shows an example relationship between the normal contribution $P_{n,g}$ of the gravity exerted on the probe as a function of the circumferential position θ of the probe 22 in the drum 14. As can be seen, the normal contribution $P_{n,g}(\theta)$ of the gravity exerted on the probe 22 during a drum rotation varies as:

$$P_{n,g}(\Theta) = - K_{mg} \sin \Theta, \qquad\qquad (1)$$

where $K_{mg}$ is a constant which depends on the weight of the probe, i.e. on the mass m of the probe and on the gravitational acceleration g of earth, and θ is the circumferential position of the probe. Because of the change in orientation and sign convention, the pressure value measured by the probe is negative at the circumferential position 90° and positive in the opposite circumferential position of 270°.

In some embodiments, the constant $K_{mg}$ and the corresponding normal contribution $P_{n,g}(\theta)$ of the gravity of a given probe 22 in a given drum 14 can be obtained by measuring the pressure values $P_{n,g}$ as the drum 14 rotates over the circumferential positions θ when the drum 14 is empty (e.g., filled with air). Such data can be recorded and stored for later use as calibration data for the given probe 22 and the given drum 14. For instance, the normal contribution $P_{n,g}(\theta)$ of gravity can be subtracted from raw pressure measurements of the probe to obtain "weight compensated" (WC) pressure values $P_{n,WC}$. When the pressure values are so weight compensated, the probe 22 can measure pressure values of 0 with a given precision when the probe 22 in an empty drum. Because the probe 22 can wear with time and its weight and surface can be reduced, it is possible to adjust the weight compensation to account for the wear of the probe 22 over time.

In some other embodiments, the probe 22 is configured to compensate its own weight when moved circumferentially as the drum 14 rotates. Accordingly, when the drum 14 is empty, the pressure values measured by such a probe are constant over the plurality of circumferential positions θ. In these embodiments, the relationship between the normal contribution of the gravity exerted on the probe as a function of the circumferential position of the probe would be null or near null for all circumferential positions θ. In these cases, the constant $K_{mg}$ and the normal contribution of gravity can thus be ignored, and the raw pressure measurements of the probe can also be considered "weight compensated" pressure values $P_{n,WC}$.

The buoyancy depends on the density D of the fresh concrete displaced by the probe 22 and on a volume V of the probe 22, and acts on the probe 22 along the vertical 20. Accordingly, the normal contribution $P_{n,b}$ of the buoyancy exerted on the probe 22 varies with its circumferential position θ. For instance, when the probe 22 is horizontally-oriented, e.g., when the probe 22 is at the circumferential position θ = 90° or θ = 270°, the normal contribution $P_{n,b}$ of the buoyancy is either maximal or minimal. In contrast, when the probe 22 is vertically-oriented, i.e. when the probe 22 is at the circumferential position θ=180°, the normal contribution $P_{n,b}$ of the buoyancy is null. As can be understood, as the probe 22 can have a high volume and as the density of the fresh concrete can be high, the normal contribution $P_{n,b}$ of buoyancy on the probe can be significant, especially when the pressure values measured by the probe 22 are weight compensated.

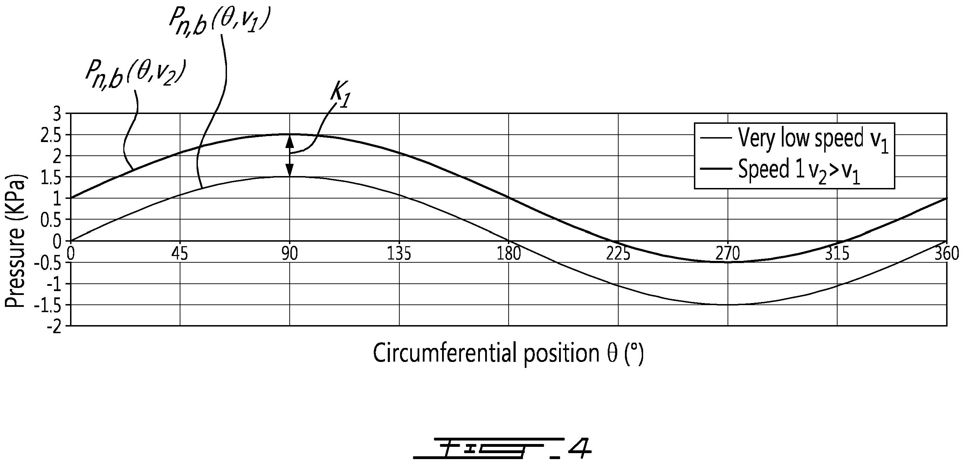

Fig. 4 shows an example relationship between the normal contribution $P_{n,b}$ of the buoyancy exerted on the probe 22 as a function of its circumferential position $\theta$ when the probe 22 is submerged into water (having a known density of 1 g/cm$^3$) when the weight of the probe 22 has been compensated as described above. As can be seen, the normal contribution $\theta$ of the buoyancy exerted on the probe 22 varies as:

$$Pn,b(\theta) = K_V \, D \sin \theta, \tag{2}$$

where $K_V$ is a constant which depends on a volume V of the probe 22, D is the density of the displaced fluid, i.e. the fresh concrete in this example, and $\theta$ is the circumferential position of the probe 22. Equation (2) assumes that there is no restriction due to the existence of any yield stress.

The constant $K_v$ associated to a given probe can be determined during a calibration step in which the probe 22 is moved inside a drum 14 filled with a fluid having a known density and during which weight compensated pressure values Pn,b($\theta$) are measured by the probe, such as the one shown in Fig. 4. In this example, the constant $K_V$ can be determined by computing $K_V = Pn,b(\theta i)/(D_{water} \sin\theta i)$ wherein $\theta i$ is any circumferential position of the probe 22. As the constant $K_V$ is associated to the construction of the probe 22, and not to the fluid in which the probe is submerged, the constant $K_V$ will remain the same regardless of the type of fluid in which the probe 22 is submerged.

The resistance exerted on the probe 22 by the fresh concrete 12 acts on the probe 22 in a normal orientation. Accordingly, the normal contribution Pn,r of the resistance exerted on the probe 22 by the fresh concrete 12 is constant for all circumferential positions $\theta$ when the probe 22 is moved in the fresh concrete 12 at any given rotation speed (e.g., v1, v2). For instance, during a rotation of the drum 14, the resistance considerably increases as the probe 22 enters in the fresh concrete 12, is constant during its passage in the fresh concrete 12, and then considerably decreases as the probe 22 exits the fresh concrete 12.

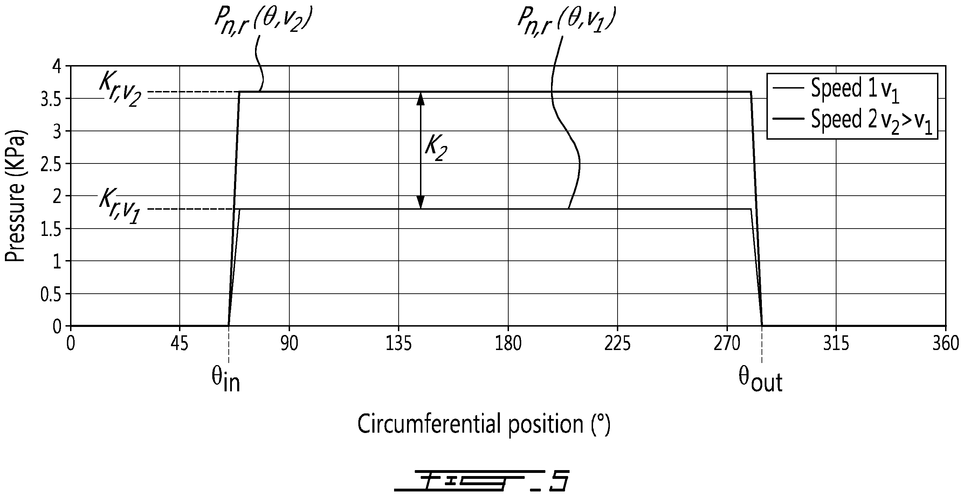

Fig. 5 shows an example relationship between the normal contribution Pn,r of the resistance exerted on the probe 22 by the fresh concrete 12 as a function of its circumferential position $\theta$ when the probe 22 is submerged into the fresh concrete 12 and when without the normal contribution Pn,g of gravity and the normal contribution Pn,b of buoyancy. As can be seen, the normal contribution Pn,r of the resistance exerted on the probe 22 by the fresh concrete 12 varies as:

$$Pn,r(\theta) = K_R \text{ for } \theta in < \theta < \theta out, \text{ and} \tag{3}$$

$$Pn,r(\theta) = 0 \text{ for } \theta < \theta in \text{ and } \theta > \theta out, \tag{4}$$

wherein $K_R$ is a constant indicative on the normal resistance exerted on the probe 22 by the fresh concrete 12 when the probe 22 is moved inside the fresh concrete 12 at a given rotation speed v, $\theta$in is the circumferential position at which the probe 22 enters the fresh concrete 12 and $\theta$out is the circumferential position at which the probe 12 exists the fresh concrete 12. The constant $K_R$ depends on the rotation speed v of the drum 14 and on a workability of the fresh concrete 12. As can be understood, $\theta$in and $\theta$out depends on the amount of fresh concrete 12 inside the drum.

Theoretically, the probe 22 can measure raw pressure values Pn,raw($\theta$) which are indicative of the normal contributions of gravity, buoyancy and resistance as follows:

$$Pn,raw \, (\theta) = Pn,g(\theta) + Pn,b(\theta) + Pn,r(\theta). \tag{5}$$

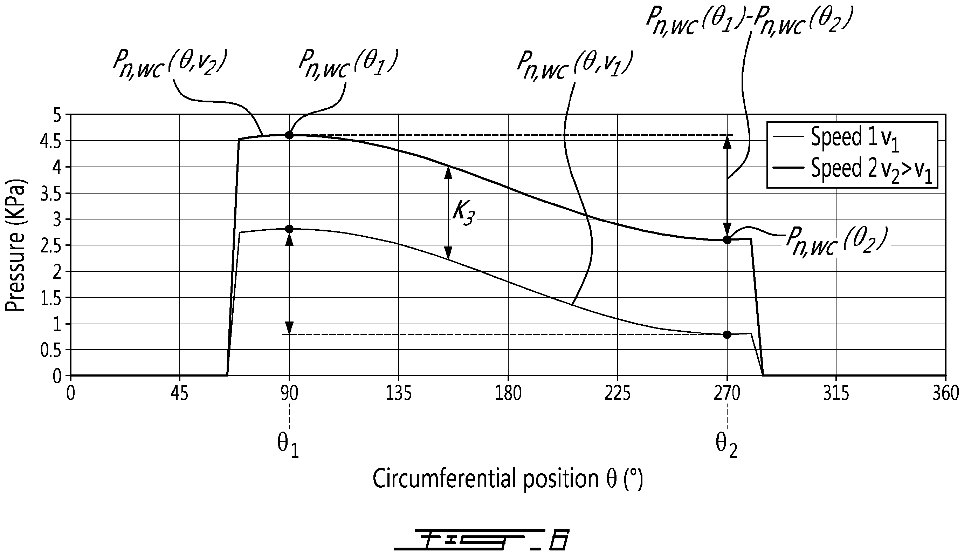

Fig. 6 shows an example relationship between the normal contributions of buoyancy and resistance as a function of the circumferential position $\theta$ when the probe is submerged into the fresh concrete 12, without the normal contribution Pn,g of gravity. As can be seen, such weight compensated pressure values $Pn,_{WC}(\theta)$ are given by:

$$Pn,_{WC}(\theta) = Pn,raw \, (\theta) - Pn,g(\theta)$$

$$= Pn,b(\theta) + Pn,r(\theta). \tag{6}$$

It was found that the difference between the pressure values taken at two different circumferential positions $\theta$ is proportional to the density value D of the fresh concrete 12.

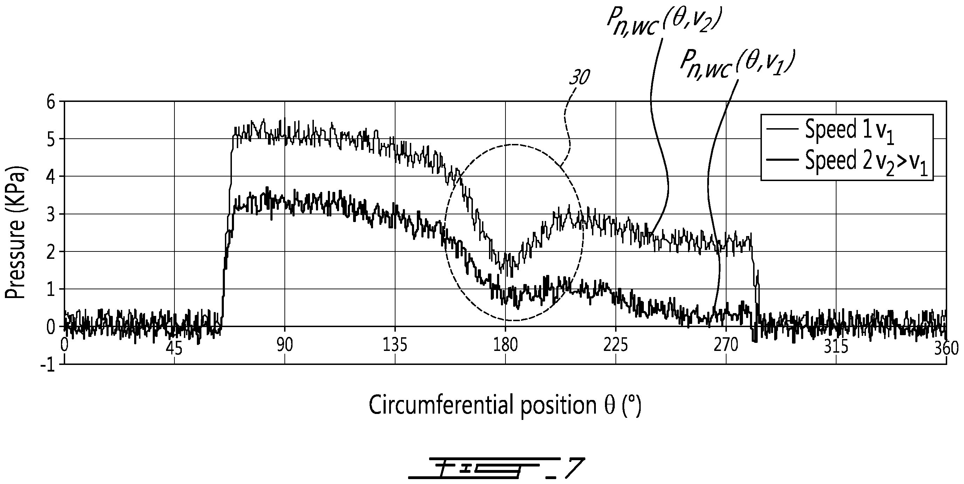

Fig. 7 shows an example of an experimental relationship between the normal contributions $Pn_{,WC}(\theta)$ of buoyancy and resistance as a function of its circumferential position when the probe is submerged into fresh concrete, without the normal contribution of gravity. One difference between the theoretical data plotted in Fig. 6 and the experimental data plotted in Fig. 7 is that the pressure values measured when the probe 22 is in the vicinity of the bottom of the drum i.e. near the circumferential position $\theta = 180°$, have some discrepancies 30 from what would be theoretically expected. These discrepancies 30 can stem from some movement of the fresh concrete 12 along the rotation axis 18 of the drum 14 due to the mixing blade action which reduce the pressure on the probe 22.

Example 1 - Determining the density of the fresh concrete using weight compensated pressure values

For instance, in one example, a first weight compensated pressure value $Pn_{,WC}(\theta1)$ is measured when the probe 22 is at a first circumferential position $\theta1$ and a second weight compensated pressure value $Pn_{,WC}(\theta2)$ is measured when the probe 22 is at a second circumferential position $\theta2$, as shown in Fig. 6.

Now, using equation (6) above, one can obtain:

$$Pn_{,wc}(\theta1) - Pn_{,wc}(\theta2) = (Pn,b(\theta1) + Pn,r(\theta1)) - (Pn,b(\theta2) + Pn,r(\theta2)),$$

or, equivalently,

$$Pn_{,wc}(\theta1) - Pn_{,wc}(\theta2) = (Pn,b(\theta1) - Pn,b(\theta2)) + (Pn,r(\theta1) - Pn,r(\theta2)).$$

As can be understood from Fig. 5 and equation (3) the quantity $Pn, r(\theta1) - Pn,r(\theta2)$ equals 0 because $Pn,r(\theta1) = Pn,r(\theta2) = K_R$, which leaves, using equation (2),

$$Pn_{,wc}(\theta1) - Pn_{,wc}(\theta2) = K_V\ D\ (\sin\theta1 - \sin\theta2). \qquad (7)$$

From equation (7), one can determine that the density value D of the fresh concrete 12 is given by:

$$D = (Pn_{,wc}(\theta1) - Pn_{,wc}(\theta2)) / (K_V(\sin\theta1 - \sin\theta2)) \qquad (8)$$

In the example shown in Fig. 6, the first circumferential position $\theta1$ is 90° and the second circumferential position $\theta2$ is 270°. Accordingly, the trigonometric factor $(\sin\theta1 - \sin\theta2)$ corresponds to 2 and the density value corresponds to the difference $(Pn_{,WC}(\theta1) - Pn_{,WC}(\theta2))$ which is compensated by a trigonometric factor corresponding to $2K_V$. As mentioned above, the constant $K_V$ depends on the volume V of the probe 22, and can be known for a given probe 22, so as to allow the determination of the density value D of the fresh concrete 12.

It is noted that in some embodiments, the trigonometric factor $(\sin\theta1 - \sin\theta2)$ may correspond to 1. For instance, it can occur when the first circumferential position $\theta1$ is 90° and the second circumferential position $\theta2$ is 180°. In such embodiments, the density value D is determined mainly based on the constant $K_V$ and on the difference $(Pn_{,WC}(\theta1) - Pn_{,WC}(\theta2))$, without considering the trigonometric factor. Indeed, even if the difference $(Pn_{,WC}(\theta1) - Pn_{,WC}(\theta2))$ were to be compensated by the trigonometric factor 1, it would have no consequence on the determined density value, as multiplying/dividing by 1 would be inconsequential. The density value D can thus be determined without necessarily compensating the difference $(Pn_{,WC}(\theta1) - Pn_{,WC}(\theta2))$ with the trigonometric factor.

Example 2 - Determining the density value D of the fresh concrete using raw pressure values (pressure values which are not weight compensated)

In one other example, a first pressure value $Pn,raw(\theta1)$ is measured when the probe 22 is at a first circumferential position $\theta1$ and a second pressure value $Pn,raw(\theta2)$ is measured when the probe 22 is at a second circumferential position $\theta2$.

Using equation (5) above, one can obtain:

$$Pn,raw\ (\Theta 1)\ -\ Pn,raw\ (\Theta 2)\ =\ Pn,g(\Theta 1)\ +\ Pn,b(\Theta 1)\ +\ Pn,r(\Theta 1)\ -\ Pn,g(\Theta 2)\ -\ Pn,b(\Theta 2)\ -\ Pn,r(\Theta 2),$$

or, equivalently,

$$Pn,raw\ (\Theta 1)\ -\ Pn,raw\ (\Theta 2)\ =\ (Pn,g(\Theta 1)\ -\ Pn,g(\Theta 2))\ +\ (Pn,b(\Theta 1)\ -\ Pn,b(\Theta 2))\ +\ (Pn,r(\Theta 1)\ -\ Pn,r(\Theta 2)).$$

As can be understood from Fig. 5, and equation (3), the quantity $Pn,r(\theta 1) - Pn,r(\theta 2)$ equals 0 because $Pn,r(\theta 1) = Pn,r(\theta 2) = K_R$, which leaves, using equations (1) and (2):

$$Pn,raw\ (\Theta 1)\ -\ Pn,raw\ (\Theta 2)\ =\ (K_V\ D\ -\ K_{mg})(\sin \Theta 1\ -\ \sin \Theta 2). \qquad (9)$$

From equation (9), one can determine that the density value D of the fresh concrete 12 is given by:

$$D = (Pn,raw\ (\Theta 1)\ -\ Pn,raw\ (\Theta 2))/(K_V(\sin \Theta 1\ -\ \sin \Theta 2))\ +\ K_{mg}/K_V \qquad (10)$$

In a case where the first circumferential position $\theta 1$ is 90° and the second circumferential position $\theta 2$ is 270°, the trigonometric factor ($\sin \theta 1 - \sin \theta 2$) yields 2 and the density value corresponds to the difference ($Pn,_{WC}(\theta 1) - Pn,_{WC}(\theta 2)$) which is compensated by a trigonometric factor corresponding to 2Kv plus a constant value based on the constants $K_{mg}$ and $K_V$. As mentioned above, the constant $K_V$ depends on the volume V of the probe 12 whereas the constant $K_{mg}$ depends on the mass m of the probe 22 and on the gravitational acceleration g on earth, which are all constant for a given probe 22, and allows the determination of the density value D of the fresh concrete 12.

As can be understood from the examples of determining the density value D of the fresh concrete 12 described above, the density value D of the fresh concrete 12 is determined based on the volume V of the probe 22 and on a difference between the first pressure value and the second pressure value being compensated by a trigonometric factor corresponding to a difference between the sinus of the first circumferential position and the sinus of the second circumferential position. That trigonometric factor can be equal to ($\sin \theta 1 - \sin \theta 2$) or to any other suitable trigonometrically equivalent factor.

As some pressure values measured when the probe 22 is in the vicinity of the bottom of the drum 14 can have some discrepancies, e.g., discrepancies 20 shown in Fig. 7, from theoretical expectations, it is generally preferred to avoid using pressure values measured when the probe 22 is in the bottom of the drum 14 in the determination of the density value D of the fresh concrete 12. In other words, none of the first and second circumferential positions corresponds to the bottom of the drum 14, e.g., $\theta$ = 180° in the example shown.

As can be understood, as the density value D of the fresh concrete 12 is proportional to the difference between the first pressure value and the second pressure value, increasing the difference between the first pressure value and the second pressure value can in turn increase the precision with which the density value D is determined.

Accordingly, in some embodiments, the first circumferential position $\theta 1$ is chosen to lie on one side of the drum relative to the vertical 20 and the second circumferential position $\theta 2$ lies on one other side of the drum relative to the vertical 20. For instance, the first circumferential position $\theta 1$ can lie between 90° and 180° whereas the second circumferential position $\theta 2$ can lie between 180° and 270°.

In these embodiments, the first circumferential position $\theta 1$ is chosen so as to be opposite to the second circumferential position $\theta 2$ with respect to the vertical 20. For instance, the first circumferential position $\theta 1$ is 90° and the second circumferential position $\theta 2$ is 270°. The first circumferential position $\theta 1$ can be 112.5° and the second circumferential position $\theta 2$ is 247.5° in another example.

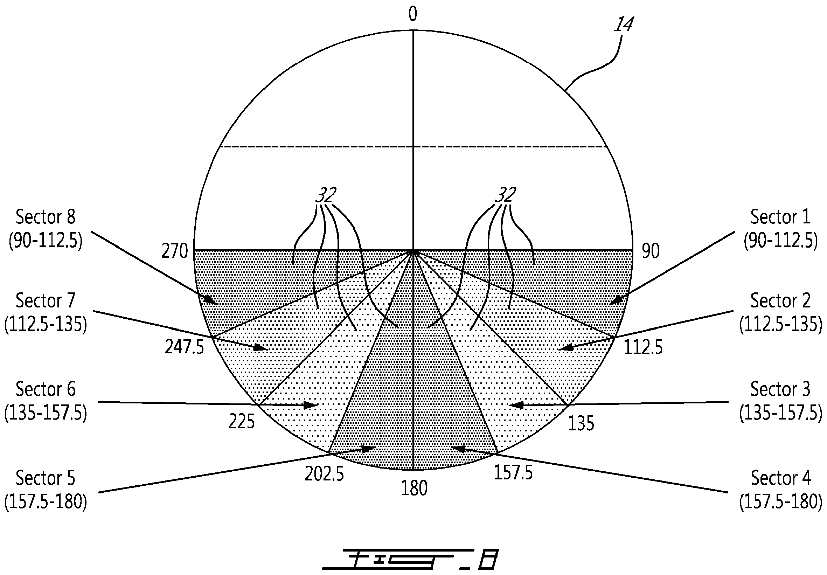

Fig. 8 shows an example of a section of the drum 14, in accordance with another embodiment. As depicted, the drum 14 is divided into a plurality of virtual circumferential ranges 32. More specifically, in this example, the drum 14 is divided into eight different circumferentially-spaced circumferential ranges which spans in the bottom hemisphere of the drum 14. As shown, the circumferential position of the first circumferential range spans between 90° and 112.5°, the circumferential position of the second circumferential range spans between 112.5° and 135°, the circumferential position of the third circumferential range spans between 135° and 157.5°, the circumferential position of the fourth circumferential range spans between 157.5° and 180°, the circumferential position of the fifth circumferential range spans between 180° and 202.5°, the circumferential position of the sixth circumferential range spans between 202.5° and 225°, the circumferential position of the seventh circumferential range spans between 225° and 247.5°, and the circumferential position of the eighth circumferential range spans between 247.5° and 270°.

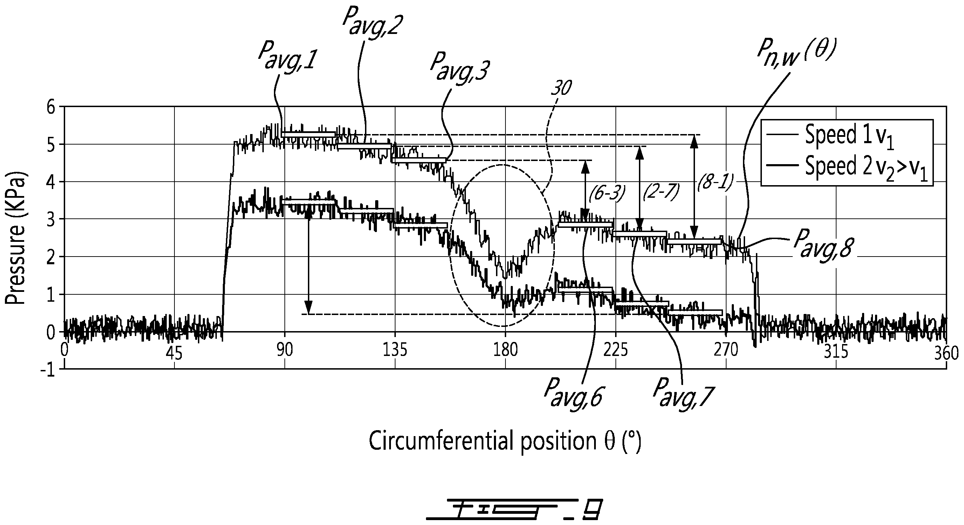

Fig. 9 shows an example of experimental weight compensated pressure values $Pn,_{WC}(\theta)$ measured by the probe 22 during one rotation of the drum 14. As it can be seen, the pressure values associated with the circumferential positions of each one of the circumferential ranges 32 can be averaged to yield averaged pressure values Pavg in each of the circumferential ranges 32.

Accordingly, in one embodiment, the first pressure value with which the density value D of the fresh concrete 12 is determined corresponds to an average of the pressure values measured when the probe 22 was moved in one of the circumferential ranges, e.g., the first circumferential range.

In another embodiment, the second pressure value with which the density value D of the fresh concrete 12 is determined corresponds to an average of the pressure values measured when the probe 22 was moved in another one of the circumferential ranges, e.g., the second, third or eighth circumferential range.

Moreover, the more the two circumferential ranges are far apart one another, the more the density value D determined can be precise. For instance, as the level of fresh concrete 12 in the drum 14 progressively lowers, the density D of the fresh concrete 12 can be determined using first and second pressure values measured correspondingly progressively closer to the bottom of the drum 14. As can be understood, due to the presence of the discrepancies 30 in the vicinity of the bottom of the drum 14, the fourth and fifth circumferential ranges can be ignored.

It was found that the difference between the first and second pressure values is generally constant regardless of the rotation speed v of the drum 14. For instance, as shown in Fig. 4, a normal contribution $Pn,b(\theta,v1)$ of the buoyancy at a first rotation speed v1 differs from a normal contribution $Pn,b(\theta,v2)$ of the buoyancy at second rotation speed v2 by a constant K1. More specifically, $Pn,b(\theta,v2) = Pn,b(\theta,v1) + K1$.

Similarly, as shown in Fig. 5, a normal contribution $Pn,r(\theta,v1)$ of the resistance exerted on the probe by the concrete when the probe is moved at the first rotation speed v1 in the fresh concrete 12 differs from a normal contribution $Pn,r(\theta,v2)$ of the resistance exerted on the probe 22 by the fresh concrete 12 when the probe 22 is moved at the second rotation speed v2 in the fresh concrete by a constant L2 for the circumferential positions between $\theta in$ and $\theta out$. More specifically, $Pn,r(\theta,v2) = Pn,r(\theta,v1) + K2$. When turned at a low rotation speed, e.g., the first rotation speed v1, it is possible to neglect the effect of water viscosity. The pressure values measured when the drum 14 rotates at the second rotation speed v2 are shifted due to viscosity effect.

Accordingly, as shown in Fig. 6, weight compensated pressure values $Pn,wc(\theta,v1)$ measured by the probe 22 when moved at the first rotation speed v1 in the fresh concrete 12 differs from weight compensated pressure values $Pn,wc(\theta,v2)$ measured by the probe when moved at the second rotation speed v2 in the fresh concrete 12 by a constant K3. More specifically, $Pn,wc(\theta,v2) = Pn,wc(\theta,v1) + K3$.

Accordingly, as shown throughout Figs. 4-7, in some embodiments, the difference between a first pressure value measured when the probe 22 is at a first circumferential position $\theta1$ and a second pressure value measured when the probe 22 is at a second circumferential position $\theta2$, during a rotation of the drum 14 at the first rotation speed v1, is similar to the difference between a first pressure value measured when the probe 22 is at a first circumferential position $\theta1$ and a second pressure value measured when the probe 22 is at a second circumferential position $\theta2$, during a rotation of the drum 14 at the second rotation speed. Indeed, it was found that the difference $(Pn,WC(\theta1,v1) - Pn,WC(\theta2,v1))$ should be similar to the difference $(Pn,WC(\theta1,v2) - Pn,WC(\theta2,v2))$ for one given type of fresh concrete .

As such, the density value D of the fresh concrete 12 can be determined either based on pressure values measured as the probe 22 moves at the first rotation speed v1 or on pressure values measured as the probe 22 moves at the second rotation speed v2. In another embodiment, it is envisaged that the density value D of the fresh concrete 12 can be determined based on a first pressure value measured when the probe 22 is a first circumferential position $\theta1$ during a rotation of the drum 14 at the first rotation speed v1 and on a second pressure value measured when the probe 22 is at a second circumferential position $\theta2$ during a rotation of the drum 14 at the second rotation speed v2, given that the either one of the constants K1, K2 and K3 above be known. One can thus calibrate the probe 22 based on a known variation of the rotation speeds. That is, the first and second pressure values need not to be measured at a same rotation speed.

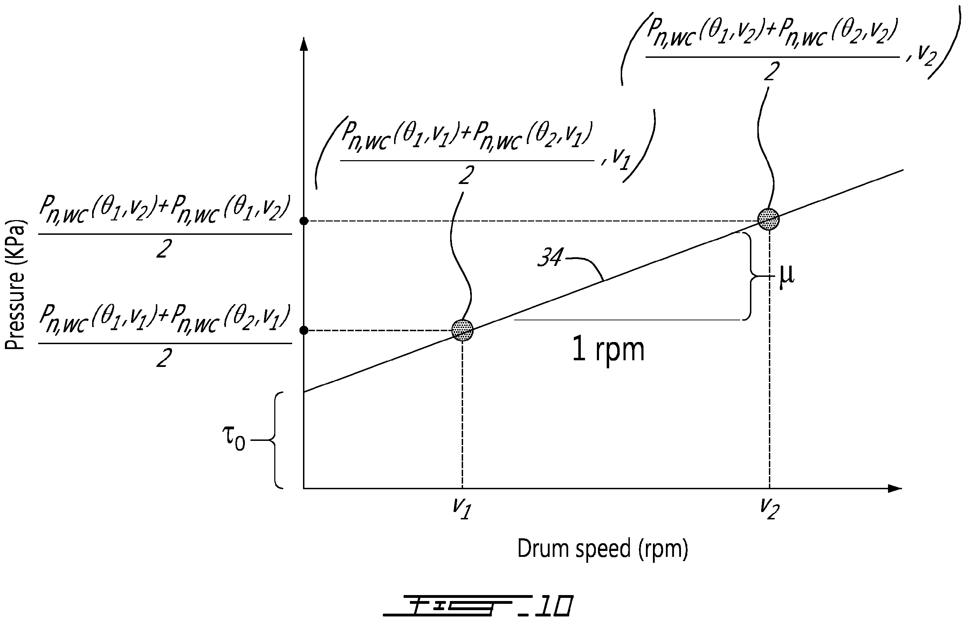

Fig. 10 shows an example of a graph showing the average of the first and second pressure values as measured when the probe is at the first and second circumferential positions $\theta1$ and $\theta2$ during rotation at different rotation speeds of the drum as function of the different rotation speeds of the drum. As shown, rheological properties of the fresh concrete 12 can be determined from the graph of Fig. 10. More specifically, the viscosity $\mu$ of the fresh concrete 12 can be determined by calculating a slope of the resulting linear relationship 34. Further, a yield $\tau0$ of the fresh concrete 12 can be determined by extrapolating the pressure value at a null rotation speed.

It is noted that using pressure values taken far away from the bottom of the drum can yield more precise rheological property measurements than measurements using pressure values taken in the vicinity of the drum because it may not be disrupted by the level of fresh concrete in the drum.

As can be understood, the examples described above and illustrated are intended to be exemplary only. For instance, the trigonometric factor which is used to compensate the difference between the first pressure value and the second pressure value can correspond to the difference between the sinus of the first circumferential position and the sinus of the second circumferential position in some embodiments whereas the trigonometric factor can alternatively correspond to the difference between the cosine of the first circumferential position and the cosine of the second circumferential position in some other embodiments. The sinus/cosine is determined based on how the circumferential positions are defined relative to the circumference of the drum. The scope is indicated by the appended claims.

**Claims**

1. A method for determining density of fresh concrete inside a drum of a mixer truck using a probe, the drum having a rotation axis being at least partially horizontally-oriented, the probe being mounted inside the drum, extending in a radial orientation of the drum and being moved circumferentially as the drum rotates, and onto which a normal pressure is imparted by resistance due to the movement of the probe in the fresh concrete by the rotation of the drum and a normal pressure contribution is imparted by buoyancy due to a volume of the probe when the probe is submerged in the fresh concrete, the method comprising:

   receiving a first pressure value indicative of a normal pressure contribution exerted on the probe by the fresh concrete at a first circumferential position of the drum during rotation of the drum;
   receiving a second pressure value indicative of a normal pressure contribution exerted on the probe by the fresh concrete at a second circumferential position during rotation of the drum, the first circumferential position being different from the second circumferential position; and
   determining a density value of the fresh concrete based on the volume of the probe, the first pressure value and the second pressure value;
   wherein the first circumferential position lies on one side of the drum relative to the vertical and the second circumferential position lies on one other side of the drum relative to the vertical,
   wherein the step of determining includes using a trigonometric factor corresponding to a difference between the sinus of the first circumferential position and the sinus of the second circumferential position, and wherein none of the first and second circumferential positions of the drum corresponds to the bottom of the drum.

2. The method of claim 1 wherein the step of determining includes compensating a difference between the first pressure value and the second pressure value by the trigonometric factor.

3. The method of claim 1 wherein the first circumferential position ranges between 90° and 135° and the second circumferential position ranges between 225° and 270° as measured from a top of the drum.

4. The method of claim 1 wherein the first circumferential position is opposite to the second circumferential position with respect to the vertical.

5. The method of claim 1 wherein the normal pressure contribution is imparted also by a weight of the probe acting on the probe, the first pressure value having been compensated by a normal contribution of the weight of the probe at the first circumferential position and the second pressure value having been compensated by a normal contribution of the weight of the probe at the second circumferential position.

6. The method of claim 1 wherein said receiving the first pressure value includes receiving a first set of pressure values from corresponding ones of a first set of circumferential positions of a given circumferential range, wherein the first pressure value is an average of the pressure values of the first set and the first circumferential position is an average of the circumferential positions of the first set inside the given circumferential range.

7. The method of claim 6 wherein said receiving the second pressure value includes receiving a second set of pressure values from corresponding ones of a second set of circumferential positions of a given circumferential range, wherein the second pressure value is an average of the pressure values of the second set and the second circumferential position is an average of the circumferential positions of the second set inside the given circumferential range.

8. The method of claim 1 wherein the first and second pressure values are measured during rotation of the drum at a first rotation speed.

9. The method of claim 8 further comprising

receiving a third pressure value indicative of a normal pressure contribution exerted on the probe by the fresh concrete at the first circumferential position of the drum during rotation of the drum at a second rotation speed different from the first rotation speed;

receiving a fourth pressure value indicative of a normal pressure contribution exerted on the probe by the fresh concrete at the second circumferential position during rotation of the drum at the second rotation speed; and

determining at least one rheological property of the fresh concrete based on the first pressure value, the second pressure value, the third pressure value and the fourth pressure value, the first rotation speed and the second rotation speed.

10. A system for determining density of fresh concrete inside a drum of a mixer truck, the drum having a rotation axis being at least partially horizontally-oriented, the system comprising:

a probe mounted inside the drum, extending in a radial orientation of the drum and being moved circumferentially as the drum rotates, and onto which a normal pressure is imparted by resistance due to the movement of the probe in the fresh concrete by the rotation of the drum and a normal pressure contribution is imparted by buoyancy due to a volume of the probe when the probe is submerged in the fresh concrete and;

a computing device communicatively coupled with the probe, the computing device being configured for performing the steps of:

receiving a first pressure value indicative of a normal pressure contribution exerted on the probe by the fresh concrete at a first circumferential position of the drum during rotation of the drum;

receiving a second pressure value indicative of a normal pressure contribution exerted on the probe by the fresh concrete at a second circumferential position during rotation of the drum, the first circumferential position being different from the second circumferential position; and

determining a density value of the fresh concrete based on the volume of the probe, the first pressure value and the second pressure value; and

a user interface communicatively coupled with the computing device, the user interface being configured to display the density value of the fresh concrete;

wherein the first circumferential position lies on one side of the drum relative to the vertical and the second circumferential position lies on one other side of the drum relative to the vertical,

wherein the step of determining includes using a trigonometric factor corresponding to a difference between the sinus of the first circumferential position and the sinus of the second circumferential position, and wherein none of the first and second circumferential positions of the drum corresponds the bottom of the drum.

11. The system of claim 10 wherein the probe is configured to compensate a weight of the probe acting of the probe when moved circumferentially as the drum rotates.

12. The system of claim 10 wherein said determining includes compensating a difference between the first pressure value and the second pressure value by the trigonometric factor.

13. The system of claim 10 wherein the first circumferential position is opposite to the second circumferential position with respect to the vertical.

14. The system of claim 10 wherein the normal pressure contribution is imparted also by a weight of the probe acting on the probe, the first pressure value having been compensated by a normal contribution of the weight of the probe at the first circumferential position and the second pressure value having been compensated by a normal contribution of the weight of the probe at the second circumferential position.

15. The system of claim 10 wherein said receiving the first pressure value includes receiving a first set of pressure values from corresponding ones of a first set of circumferential positions of a given circumferential range, wherein the first pressure value is an average of the pressure values of the first set and the first circumferential position is an average of the circumferential positions of the first set inside the given circumferential range.

**Patentansprüche**

1. Verfahren zum Bestimmen einer Dichte von Frischbeton innerhalb einer Trommel eines Betonmischwagens unter Verwendung einer Sonde, wobei die Trommel eine Drehachse aufweist, die wenigstens teilweise horizontal ausgerichtet ist, wobei die Sonde innerhalb der Trommel montiert ist, sich in einer radialen Ausrichtung der Trommel erstreckt und in Umfangsrichtung bewegt wird, weil sich die Trommel dreht, und der ein Normaldruck durch einen Widerstand aufgrund der Bewegung der Sonde in dem Frischbeton durch die Drehung der Trommel verliehen wird und ein Normaldruckbeitrag durch Auftrieb aufgrund eines Volumens der Sonde verliehen wird, wenn die Sonde in den Frischbeton eingetaucht ist, wobei das Verfahren Folgendes umfasst:

Empfangen eines ersten Druckwerts, der einen Normaldruckbeitrag angibt, der auf die Sonde durch den Frischbeton an einer ersten Umfangsposition der Trommel während der Drehung der Trommel ausgeübt wird;
Empfangen eines zweiten Druckwerts, der einen Normaldruckbeitrag angibt, der auf die Sonde durch den Frischbeton an einer zweiten Umfangsposition während der Drehung der Trommel ausgeübt wird, wobei sich die erste Umfangsposition von der zweiten Umfangsposition unterscheidet; und
Bestimmen eines Dichtewertes des Frischbetons basierend auf dem Volumen der Sonde, dem ersten Druckwert und dem zweiten Druckwert;
wobei die erste Umfangsposition auf einer Seite der Trommel relativ zu der Vertikalen liegt und die zweite Umfangsposition auf einer anderen Seite der Trommel relativ zu der Vertikalen liegt,
wobei der Schritt des Bestimmens ein Verwenden eines trigonometrischen Faktors beinhaltet, der einer Differenz zwischen dem Sinus der ersten Umfangsposition und dem Sinus der zweiten Umfangsposition entspricht, und
wobei keine der ersten und der zweiten Umfangsposition der Trommel dem Boden der Trommel entspricht.

2. Verfahren nach Anspruch 1, wobei der Schritt des Bestimmens ein Kompensieren einer Differenz zwischen dem ersten Druckwert und dem zweiten Druckwert durch den trigonometrischen Faktor beinhaltet.

3. Verfahren nach Anspruch 1, wobei die erste Umfangsposition zwischen 90° und 135° reicht und die zweite Umfangsposition zwischen 225° und 270° reicht, gemessen von einer Oberseite der Trommel.

4. Verfahren nach Anspruch 1, wobei sich die erste Umfangsposition gegenüber der zweiten Umfangsposition hinsichtlich der Vertikalen befindet.

5. Verfahren nach Anspruch 1, wobei der Normaldruckbeitrag ebenso durch ein Gewicht der Sonde verliehen wird, das auf die Sonde wirkt, wobei der erste Druckwert durch einen Normalbeitrag des Gewichts der Sonde an der ersten Umfangsposition kompensiert worden ist und der zweite Druckwert durch einen Normalbeitrag des Gewichts der Sonde an der zweiten Umfangsposition kompensiert worden ist.

6. Verfahren nach Anspruch 1, wobei das Empfangen des ersten Druckwerts das Empfangen eines ersten Satzes von Druckwerten von entsprechenden eines ersten Satzes von Umfangspositionen eines gegebenen Umfangsbereichs beinhaltet, wobei der erste Druckwert ein Durchschnitt der Druckwerte des ersten Satzes ist und die erste Umfangsposition ein Durchschnitt der Umfangspositionen des ersten Satzes innerhalb des gegebenen Umfangsbereichs ist.

7. Verfahren nach Anspruch 6, wobei das Empfangen des zweiten Druckwerts das Empfangen eines zweiten Satzes von Druckwerten von entsprechenden eines zweiten Satzes von Umfangspositionen eines gegebenen Umfangsbereichs beinhaltet, wobei der zweite Druckwert ein Durchschnitt der Druckwerte des zweiten Satzes ist und die zweite Umfangsposition ein Durchschnitt der Umfangspositionen des zweiten Satzes des gegebenen Umfangsbereichs ist.

8. Verfahren nach Anspruch 1, wobei der erste und der zweite Druckwert während der Drehung der Trommel mit einer ersten Drehzahl gemessen werden.

9. Verfahren nach Anspruch 8, das ferner Folgendes umfasst:

Empfangen eines dritten Druckwerts, der einen Normaldruckbeitrag angibt, der auf die Sonde durch den Frischbeton an der ersten Umfangsposition der Trommel während der Drehung der Trommel mit einer zweiten Drehzahl ausgeübt wird, die sich von der ersten Drehzahl unterscheidet;
Empfangen eines vierten Druckwerts, der einen Normaldruckbeitrag angibt, der auf die Sonde durch den Frischbeton an der zweiten Umfangsposition der Trommel während der Drehung der Trommel mit der zweiten Drehzahl ausgeübt wird; und
Bestimmen wenigstens einer rheologischen Eigenschaft des Frischbetons basierend auf dem ersten Druckwert, dem zweiten Druckwert, dem dritten Druckwert und dem vierten Druckwert, der ersten Drehzahl und der zweiten Drehzahl.

**10.** System zum Bestimmen der Dichte von Frischbeton innerhalb einer Trommel eines Betonmischwagens, wobei die Trommel eine Drehachse aufweist, die wenigstens teilweise horizontal ausgerichtet ist, wobei das System Folgendes umfasst:

eine Sonde, die innerhalb der Trommel montiert ist, sich in einer radialen Ausrichtung der Trommel erstreckt und in Umfangsrichtung bewegt wird, weil sich die Trommel dreht, und der ein Normaldruck durch einen Widerstand aufgrund der Bewegung der Sonde in dem Frischbeton durch die Drehung der Trommel verliehen wird und ein Normaldruckbeitrag durch Auftrieb aufgrund eines Volumens der Sonde verliehen wird, wenn die Sonde in den Frischbeton eingetaucht ist; und
eine Rechenvorrichtung, die mit der Sonde kommunikativ gekoppelt ist, wobei die Rechenvorrichtung zum Durchführen der folgenden Schritte konfiguriert ist:

Empfangen eines ersten Druckwerts, der einen Normaldruckbeitrag angibt, der auf die Sonde durch den Frischbeton an einer ersten Umfangsposition der Trommel während der Drehung der Trommel ausgeübt wird;
Empfangen eines zweiten Druckwerts, der einen Normaldruckbeitrag angibt, der auf die Sonde durch den Frischbeton an einer zweiten Umfangsposition während der Drehung der Trommel ausgeübt wird, wobei sich die erste Umfangsposition von der zweiten Umfangsposition unterscheidet; und
Bestimmen eines Dichtewertes des Frischbetons basierend auf dem Volumen der Sonde, dem ersten Druckwert und dem zweiten Druckwert; und
eine Benutzeroberfläche, die mit der Rechenvorrichtung kommunikativ gekoppelt ist, wobei die Benutzeroberfläche konfiguriert ist, um den Dichtewert des Frischbetons anzuzeigen;
wobei die erste Umfangsposition auf einer Seite der Trommel relativ zu der Vertikalen liegt und die zweite Umfangsposition auf einer anderen Seite der Trommel relativ zu der Vertikalen liegt,
wobei der Schritt des Bestimmens ein Verwenden eines trigonometrischen Faktors beinhaltet, der einer Differenz zwischen dem Sinus der ersten Umfangsposition und dem Sinus der zweiten Umfangsposition entspricht, und wobei keine der ersten und der zweiten Umfangsposition der Trommel dem Boden der Trommel entspricht.

**11.** System nach Anspruch 10, wobei die Sonde konfiguriert ist, um ein Gewicht der Sonde zu kompensieren, das auf die Sonde wirkt, wenn sie in Umfangsrichtung bewegt wird, weil sich die Trommel dreht.

**12.** System nach Anspruch 10, wobei das Bestimmen das Kompensieren einer Differenz zwischen dem ersten Druckwert und dem zweiten Druckwert durch den trigonometrischen Faktor beinhaltet.

**13.** System nach Anspruch 10, wobei sich die erste Umfangsposition gegenüber der zweiten Umfangsposition hinsichtlich der Vertikalen befindet.

**14.** System nach Anspruch 10, wobei der Normaldruckbeitrag ebenso durch ein Gewicht der Sonde verliehen wird, das auf die Sonde wirkt, wobei der erste Druckwert durch einen Normalbeitrag des Gewichts der Sonde an der ersten Umfangsposition kompensiert worden ist und der zweite Druckwert durch einen Normalbeitrag des Gewichts der Sonde an der zweiten Umfangsposition kompensiert worden ist.

**15.** System nach Anspruch 10, wobei das Empfangen des ersten Druckwerts das Empfangen eines ersten Satzes von Druckwerten von entsprechenden eines ersten Satzes von Umfangspositionen eines gegebenen Umfangsbereichs beinhaltet, wobei der erste Druckwert ein Durchschnitt der Druckwerte des ersten Satzes ist und die erste Umfangsposition ein Durchschnitt der Umfangspositionen des ersten Satzes innerhalb des gegebenen Umfangsbereichs ist.

**Revendications**

**1.** Procédé de détermination de la densité de béton frais à l'intérieur d'un tambour d'un camion malaxeur à l'aide d'une sonde, le tambour ayant un axe de rotation étant au moins partiellement orienté horizontalement, la sonde étant montée à l'intérieur du tambour, s'étendant dans une orientation radiale du tambour et étant déplacée circonférentiellement lorsque le tambour tourne, et sur laquelle une pression normale est appliquée par une résistance due au mouvement de la sonde dans le béton frais par la rotation du tambour et une contribution de pression normale est appliquée par la flottabilité due à un volume de la sonde lorsque la sonde est immergée dans le béton frais, le procédé comprenant :

la réception d'une première valeur de pression indiquant une contribution de pression normale exercée sur la sonde par le béton frais au niveau d'une première position circonférentielle du tambour pendant la rotation du tambour ;

la réception d'une deuxième valeur de pression indiquant une contribution de pression normale exercée sur la sonde par le béton frais au niveau d'une seconde position circonférentielle pendant la rotation du tambour, la première position circonférentielle étant différente de la seconde position circonférentielle ; et

la détermination d'une valeur de densité du béton frais sur la base du volume de la sonde, de la première valeur de pression et de la deuxième valeur de pression ;

la première position circonférentielle se trouvant sur un côté du tambour par rapport à la verticale et la seconde position circonférentielle se trouvant sur un autre côté du tambour par rapport à la verticale,

l'étape de détermination comportant l'utilisation d'un facteur trigonométrique correspondant à une différence entre le sinus de la première position circonférentielle et le sinus de la seconde position circonférentielle, et aucune des première et seconde positions circonférentielles du tambour ne correspondant au fond du tambour.

2. Procédé selon la revendication 1, l'étape de détermination comportant la compensation d'une différence entre la première valeur de pression et la deuxième valeur de pression par le facteur trigonométrique.

3. Procédé selon la revendication 1, la première position circonférentielle étant comprise entre 90° et 135° et la seconde position circonférentielle étant comprise entre 225° et 270° tel que mesuré à partir d'un sommet du tambour.

4. Procédé selon la revendication 1, la première position circonférentielle étant à l'opposé de la seconde position circonférentielle par rapport à la verticale.

5. Procédé selon la revendication 1, la contribution de pression normale étant également appliquée par un poids de la sonde agissant sur la sonde, la première valeur de pression ayant été compensée par une contribution normale du poids de la sonde au niveau de la première position circonférentielle et la deuxième valeur de pression ayant été compensée par une contribution normale du poids de la sonde au niveau de la seconde position circonférentielle.

6. Procédé selon la revendication 1, ladite réception de la première valeur de pression comportant la réception d'un premier ensemble de valeurs de pression à partir de valeurs correspondantes d'un premier ensemble de positions circonférentielles d'une plage circonférentielle donnée, la première valeur de pression étant une moyenne des valeurs de pression du premier ensemble et la première position circonférentielle étant une moyenne des positions circonférentielles du premier ensemble dans de la plage circonférentielle donnée.

7. Procédé selon la revendication 6, ladite réception de la deuxième valeur de pression comportant la réception d'un second ensemble de valeurs de pression à partir de valeurs de pression correspondantes d'un second ensemble de positions circonférentielles d'une plage circonférentielle donnée, la deuxième valeur de pression étant une moyenne des valeurs de pression du second ensemble et la seconde position circonférentielle étant une moyenne des positions circonférentielles du second ensemble dans la plage circonférentielle donnée.

8. Procédé selon la revendication 1, les première et deuxième valeurs de pression étant mesurées pendant la rotation du tambour au niveau d'une première vitesse de rotation.

9. Procédé selon la revendication 8, comprenant en outre

la réception d'une troisième valeur de pression indiquant une contribution de pression normale exercée sur la sonde par le béton frais au niveau de la première position circonférentielle du tambour pendant la rotation du tambour à une seconde vitesse de rotation différente de la première vitesse de rotation ;

la réception d'une quatrième valeur de pression indiquant une contribution de pression normale exercée sur la sonde par le béton frais au niveau de la seconde position circonférentielle pendant la rotation du tambour à la seconde vitesse de rotation ; et

la détermination d'au moins une propriété rhéologique du béton frais sur la base de la première valeur de pression, de la deuxième valeur de pression, de la troisième valeur de pression et de la quatrième valeur de pression, de la première vitesse de rotation et de la seconde vitesse de rotation.

**10.** Système de détermination de la densité de béton frais à l'intérieur d'un tambour d'un camion malaxeur, le tambour ayant un axe de rotation étant au moins partiellement orienté horizontalement, le système comprenant :

une sonde montée à l'intérieur du tambour, s'étendant dans une orientation radiale du tambour et étant déplacée circonférentiellement lorsque le tambour tourne, et sur laquelle une pression normale est appliquée par une résistance due au mouvement de la sonde dans le béton frais par la rotation du tambour et une contribution de pression normale est appliquée par la flottabilité due à un volume de la sonde lorsque la sonde est immergée dans le béton frais et ;

un dispositif informatique couplé en communication avec la sonde, le dispositif informatique étant configuré pour effectuer les étapes consistant à :

réceptionner une première valeur de pression indiquant une contribution de pression normale exercée sur la sonde par le béton frais au niveau d'une première position circonférentielle du tambour pendant la rotation du tambour ;

réceptionner une deuxième valeur de pression indiquant une contribution de pression normale exercée sur la sonde par le béton frais au niveau d'une seconde position circonférentielle pendant la rotation du tambour, la première position circonférentielle étant différente de la seconde position circonférentielle ; et

déterminer une valeur de densité du béton frais sur la base du volume de la sonde, de la première valeur de pression et de la deuxième valeur de pression ; et

une interface utilisateur couplée en communication avec le dispositif informatique, l'interface utilisateur étant configurée pour afficher la valeur de densité du béton frais ;

la première position circonférentielle se trouvant sur un côté du tambour par rapport à la verticale et la seconde position circonférentielle se trouvant sur un autre côté du tambour par rapport à la verticale,

l'étape de détermination comportant l'utilisation d'un facteur trigonométrique correspondant à une différence entre le sinus de la première position circonférentielle et le sinus de la seconde position circonférentielle, et aucune des première et seconde positions circonférentielles du tambour ne correspondant au fond du tambour.

**11.** Système selon la revendication 10, la sonde étant conçue pour compenser un poids de la sonde agissant sur la sonde quand elle est déplacée circonférentiellement lorsque le tambour tourne.

**12.** Système selon la revendication 10, ladite détermination comportant la compensation d'une différence entre la première valeur de pression et la deuxième valeur de pression par le facteur trigonométrique.

**13.** Système selon la revendication 10, la première position circonférentielle étant à l'opposé de la seconde position circonférentielle par rapport à la verticale.

**14.** Système selon la revendication 10, la contribution de pression normale étant également appliquée par un poids de la sonde agissant sur la sonde, la première valeur de pression ayant été compensée par une contribution normale du poids de la sonde au niveau de la première position circonférentielle et la deuxième valeur de pression ayant été compensée par une contribution normale du poids de la sonde au niveau de la seconde position circonférentielle.

**15.** Système selon la revendication 10, ladite réception de la première valeur de pression comportant la réception d'un premier ensemble de valeurs de pression à partir de valeurs de pression correspondantes d'un premier ensemble de positions circonférentielles d'une plage circonférentielle donnée, la première valeur de pression étant une moyenne des valeurs de pression du premier ensemble et la première position circonférentielle étant une moyenne des positions circonférentielles du premier ensemble dans la plage circonférentielle donnée.

FIG. 1

FIG. 2

FIG - 3

FIG.4

FIG. 5

FIG. 6

EP 3 658 887 B1

$P_{n,wc}(\theta, v_2)$

$P_{n,wc}(\theta, v_1)$

30

6

5

4

3

2

1

0

-1

Pressure (KPa)

Speed 1 $v_1$
Speed 2 $v_2 > v_1$

0   45   90   135   180   225   270   315   360

Circumferential position $\theta$ (°)

FIG. 7

0

*14*

Sector 8
(90-112.5)

Sector 7
(112.5-135)

Sector 6
(135-157.5)

Sector 5
(157.5-180)

Sector 1
(90-112.5)

Sector 2
(112.5-135)

Sector 3
(135-157.5)

Sector 4
(157.5-180)

*32*

*32*

270

247.5

225

202.5

90

112.5

135

157.5

180

FIG. 8

Circumferential position θ (°)

FIG. 9

EP 3 658 887 B1

$$\left(\frac{P_{n,wc}(\theta_1,v_2)+P_{n,wc}(\theta_2,v_2)}{2},v_2\right)$$

$$\left(\frac{P_{n,wc}(\theta_1,v_1)+P_{n,wc}(\theta_2,v_1)}{2},v_1\right)$$

Pressure (KPa)

$$\frac{P_{n,wc}(\theta_1,v_2)+P_{n,wc}(\theta_1,v_2)}{2}$$

34

μ

$$\frac{P_{n,wc}(\theta_1,v_1)+P_{n,wc}(\theta_2,v_1)}{2}$$

1 rpm

$\tau_0$

$v_1$

$v_2$

Drum speed (rpm)

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014138968 A

- WO 2011042880 A